# EUROPÄISCHE PATENTSCHRIFT

(11) **EP 0 827 714 B1**
(45) Veröffentlichungstag und Bekanntmachung des Hinweises auf die Patenterteilung: **02.05.2002**
(21) Anmeldenummer: 97115066.9
(22) Anmeldetag: 30.08.1997
(51) Int. Cl.: A61B 5/0484, A61B 5/0478, A61B 5/12

(54) **Gerät zur Ableitung akustisch evozierter Gehirnpotentiale**
Device for the derivation of acoustically evoked cerebral potentials
Dispositif pour la dérivation des potentiels cérebraux acoustiquement provoqués

(30) Priorität: 07.09.1996 DE 29615656 U
(43) Veröffentlichungstag der Anmeldung: 11.03.1998
(60) Teilanmeldung: 02004504.3
(73) Patentinhaber: Finkenzeller, Peter, Prof. Dr. rer. nat., 91054 Erlangen (DE); Seipl, Johann, 81547 München (DE); Kammermeier, Heike, 89438 Eppisburg (DE)
(72) Erfinder: Finkenzeller, Peter,Prof. Dr. rer.nat., 91054 Erlangen (DE); Kammermeier-Blessing, Claudia, Dipl.-Oec. (Univ.), 89438 Holzheim-Eppisburg (DE)
(74) Vertreter: Gallo, Wolfgang, Dipl.-Ing. (FH)

(56) Entgegenhaltungen:
- EP-A- 0 541 315
- US-A- 3 735 753
- US-A- 3 998 213
- US-A- 4 462 411
- US-A- 4 537 198

## Beschreibung

Die Ableitung akustisch evozierter elektrischer Gehirnpotentiale ist ein bekanntes audiometrisches Diagnoseverfahren zur Prüfung des Hörvermögens und zur Beurteilung verschiedener Ursachen von Hörschäden ohne aktive Mitwirkung des Probanden.

Dieses Verfahren wird in der Fachwelt als ERA (Electric Response Audiometry) bzw. BERA (Brainstem Electric Response Audiometry) bzw. Hirnstamm-Audiometrie bezeichnet. Einsatzgebiete dieses Verfahrens sind beispielsweise die Durchführung erster Hörtests bei Neugeborenen, die Prüfung der Hörfähigkeit von Säuglingen oder auch von bewußtlosen Personen, wie z.B. Unfallopfer, und die Diagnose von neurologischen Erkrankungen, z.B. Akustikus-Neurinomen. Auch intraoperative Hörfähigkeitsprüfungen sind mit diesem Verfahren möglich.

Die Auslösung von elektrischen Gehirnpotentialen erfolgt dabei durch akustische Stimulation des Ohres über Luft- oder Knochenleitung. Dazu verwendet man üblicherweise Kopfhörer.

Die dadurch vom Hirnstamm erzeugten elektrischen Signale werden über am Kopf angebrachte Elektroden abgeleitet. Üblicherweise werden drei Elektroden verwendet, nämlich eine Elektrode zur Festlegung des Bezugspotentials und zwei aktive Elektroden zur Ableitung der akustisch evozierten elektrischen Signale an zwei verschiedenen Stellen des Kopfes.

Die akustische Stimulation des Ohres erfolgt dabei in Form von Klick-Reizen oder zur unmittelbaren Bestimmung der Hörschwelle aus einer rasch aufeinanderfolgenden Serie von Klicks mit aufsteigendem Pegel. Der Hirnstamm erzeugt auf jeden Klick Potentialwellen, die nach Erfassung und Ableitung über die Elektroden gemittelt werden.

Bisher werden die Elektroden üblicherweise angeklebt oder mechanisch oder anderweitig einzeln am Kopf fixiert. Dies ist zum einen zeitaufwendig, zum anderen belastet es den Patienten. Wegen der Kabelverbindungen besteht auch die Gefahr des falschen Zusammensteckens. Außerdem ist die Anordnung elektrischen Einstreuungen durch Fremdfelder unterworfen, was wegen der äußerst kleinen abzuleitenden Signalpotentiale kritisch ist. Hinzu kommt, daß das Ankleben und nachfolgende Wiederablösen der Elektroden an der empfindlichen Haut von Neugeborenen bzw. Säuglingen problematisch ist.

Aus der US-A-4 462 411 ist eine Anordnung mit den Merkmalen des Oberbegriffs des Anspruchs 1 bekannt. Dort umfaßt die Anordnung neben den am kopf anzubringenden Elektroden einen kopfhörer mit zwei über einen relativ starren Bügel verbundenen Hörmuscheln.

Der Erfindung liegt daher die Aufgabe zugrunde, ein Gerät zu schaffen, mit welchem eine verbesserte Ableitung der Gehirnpotentiale möglich ist.

Diese Aufgabe wird gemäß der Erfindung durch das in den Ansprüchen angegebene Gerät gelöst.

Bei dem erfindungsgemäßen Gerät sind die Elektroden zu einer in sich relativ starren Anordnung zusammengefaßt, die vom Operator mit der Hand an den Kopf des Probanden gehalten oder anderweitig als Ganzes am Kopf fixiert wird, und ein als Reizgeber dienender akustischer Wandler ist am Rahmen angeordnet. Das Gerät beaufschlagt den Reizgeber mit entsprechenden Signalen, um die zur akustischen Reizung dienende Klickfolge zu erzeugen, und mit der Elektrodenanordnung werden die dadurch evozierten elektrischen Gehirnpotentiale abgeleitet und in dem Gerät ausgewertet. Das erfindungsgemäße Gerät ist insbesondere bei Säuglingen von Vorteil, bei denen die Untersuchung üblicherweise stattfindet, wenn sie schlafen, da insbesondere das Anbringen und die Entfernung der Elektroden keiner besonderen Manipulation bedarf und so die Messung am Probanden, z.B. einem Säugling, innerhalb wesentlich kürzerer Zeit als bisher üblich vollständig erfolgt.

Die Zusammenfassung mehrerer Elektroden zu einer in sich relativ starren Anordnung, die als Ganzes am Kopf einer Person angeordnet werden kann, ist an sich aus der US-PS 4 706 679 zum Zwecke der Elektroenzephalographie bekannt. Dort ist ein Rahmen mit mehreren federnden Schenkeln vorgesehen, die an ihren Enden Elektroden tragen, und elektrische Gehirnpotentiale zu erfassen. Bei der bekannten Anordnung ist der Rahmen insbesondere dafür vorgesehen, an der Lehne eines Patientenstuhles angebracht zu werden, in dem sich der Patient für das Elektroenzephalogramm hineinlegt. Jedoch liefert dieser Stand der Technik keine Anregung zur Durchführung der Hirnstamm-Audiometrie unter Anwendung derart zusammengefaßter Elektroden in Kombination mit einem akustischen Reizgeber und einer zur Reizsignalerzeugung und zur Ableitung und Auswertung der evozierten Hirnstammsignale dienenden Signalgeber/Signalauswerteeinheit.

Vorzugsweise enthält die Elektrodeneinheit auch den als Reizgeber dienenden akustischen Wandler in Form eines Lautsprechers oder eines Knochenleitungs-Schallgebers als damit integrierte Komponente.

Bei dem erfindungsgemäßen Gerät kann die Elektrodeneinheit auch gleich den EEG-Verstärker als angebaute Komponente aufweisen, so daß ein minimaler Leitungsweg von den Ableitelektroden zum EEG-Verstärker gegeben und damit die Möglichkeit der Störpotentialeinstrahlung minimiert ist.

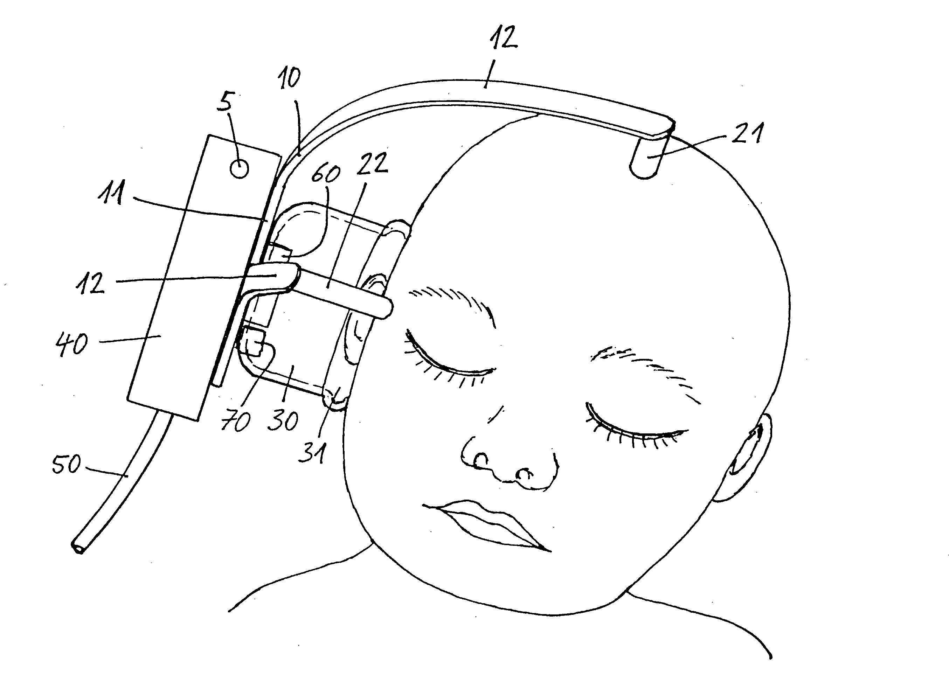

Das erfindungsgemäße Gerät wird nachstehend eines Ausführungsbeispiels unter Bezugnahme auf die anliegende Zeichnung kurz in seinen wesentlichen Einzelheiten beschrieben. Die Zeichnung zeigt eine Ausführungsform des Geräts mit an der Elektrodeneinheit integriert angeordneter Lautsprecherkapsel.

Das Gerät besteht aus einem Rahmen 10 mit einem Zentralstück 11 und mehreren Armen 12, die an ihren Enden Elektroden 21, 22 tragen, einer am Zentralstück 11 des Rahmens befestigten Hörkapsel 30, und einem ebenfalls am Zentralstück 11 des Rahmens auf dessen von der Hörkapsel abgewandten Seite angebauten EEG-Verstärker 40.

Ein Kabel 50 verbindet das Gerät mit dem übrigen Teil des für die Hirnstamm-Audiometrie verwendeten Audiometers, das die Klicks zur akustischen Stimulation des Ohres erzeugt und die abgeleiteten Hirnstamm-Potentiale verarbeitet und auswertet. Bei dem Ausführungsbeispiel ist ein einziges Kabel 50 dargestellt, das sowohl eine Leitung zur Zufuhr der elektrischen Klick-Signale als auch eine Leitung zur Übertragung der vom EEG-Verstärker 40 vorverstärkten Hirnstamm-Potentiale enthalten kann. Selbstverständlich können dazu auch getrennte Kabel oder drahtlose Übertragungswege verwendet werden.

Die Hörkapsel 30 besteht vorzugsweise aus transparentem Kunststoff und weist zweckmäßigerweise einen weichen Randwulst 31 zum dichten Anlegen an den das Ohr umgebenden Kopfbereich auf. In der Hörkapsel 30 ist ein elektroakustischer Schallwandler 60, also ein Lautsprecher, eingebaut. Statt der Hörkapsel 30 mit dem Lautsprecher 60 oder zusätzlich hierzu kann das Gerät mit einem Knochenleitungshörer ausgestattet sein, so daß die akustische Stimulation des Ohres je nachdem über Luftleitung oder über Knochenleitung erfolgen kann.

Die im Hirnstamm aufgrund der akustischen Stimulation des Ohres erzeugten elektrischen Potentiale werden mittels der Elektroden an den Armen 12 des Rahmens 10 abgenommen. Man arbeitet üblicherweise mit drei Elektroden, nämlich einer Bezugselektrode zur Festlegung eines Bezugspotentials, und zwei ableitenden Elektroden. Die Bezugselektrode wird dabei vorderhalb des Ohres mit dem Kopf in Berührung gebracht, und von den beiden ableitenden Elektroden wird eine hinterhalb des Ohres und die andere im Scheitelbereich mit dem Kopf in Berührung gebracht.

Bei dem Ausführungsbeispiel ist die Elektrode 21 die die Hirnstamm-Potentiale im Scheitelbereich abnehmende Ableitungselektrode und die Elektrode 22 die Bezugselektrode. Die zweite, hinter dem Ohr am Kopf anliegende Ableitungselektrode ist in der Zeichnung durch die Hörkapsel 30 verdeckt und nicht sichtbar.

Beim dargestellten Ausführungsbeispiel sind alle Elektroden außerhalb der Hörkapsel 30 angeordnet. Es ist aber auch möglich, die Bezugselektrode und die hinter dem Ohr zu plazierende Ableitungselektrode innerhalb der dann entsprechend groß ausgebildeten Hörkapsel 30 anzuordnen.

Die Hörkapsel 30 besteht, wie schon gesagt, vorzugsweise aus transparentem Material, so daß von außen sowohl das überdeckte Ohr als auch der in der Kapsel eingebaute Lautsprecher 60 sichtbar ist und so vom Operator verifiziert werden kann, daß die Hörkapsel 30 richtig positioniert ist, nämlich so, daß die Abstrahlung des Lautsprechers 60 tatsächlich in den Gehörgang des überdeckten Ohr erfolgt.

Die Hörkapsel 30 mit einem Ohrkissen hat den Vorteil, daß die Stimulation des Ohres über den Lautsprecher 60 unter Abschirmung von Umgebungsgeräuschen erfolgt. Die Verwendung einer Hörkapsel 30 erlaubt es außerdem, in der Hörkapsel 30 zusätzlich ein Mikrofon 70 unterzubringen, welches den Schalldruck in der Hörkapsel mißt. Dadurch kann bei der Auswertung das dem Ohr dargebotene akustische Signal als Istwert-Signal überprüft und direkt in zeitliche und quantitative Relation mit den als Reaktion erfaßten Hirnstamm-Potentialen gesetzt werden. Zugleich ist dadurch auch der Einfluß etwaiger Umgebungsgeräusche durch die Hörkapsel 30 hindurch meßbar.

Die Hörkapsel 30 ist in Form und Orientierung selbstverständlich der länglichen Form des Ohres und seiner räumlichen Orientierung mit Bezug auf die Meßpunkte der Hirnstamm-Potentiale angepaßt. Damit das Gerät sowohl für das linke Ohr wie auch für das rechte Ohr benutzt werden kann, ist die Hörkapsel 30 vorzugsweise drehbar am Rahmen 10 angebracht, so daß sich ihre relative Orientierung mit Bezug auf den Rahmen entsprechend den Bedürfnissen für das rechte Ohr bzw. für das linke Ohr wahlweise anpassen läßt.

Statt einer Hörkapsel kann auch ein knochenleitungshörer als Reizgeber verwendet werden.

In der Praxis wird man die Elektrodeneinheit in verschiedenen Standardgrößen zur Verwendung bei Säuglingen, Kindern bzw. erwachsenen Probanden ausbilden.

Anstelle lediglich des EEG-Verstärkers 40 kann auch die komplette Meßeinrichtung für die abgeleiteten Gehirnpotentiale am Rahmen der Elektrodeneinheit 2 angeordnet sein. Die Signalübertragung zwischen der Elektrodeneinheit 2 und der Signalgeber/Auswerteeinheit 1 kann statt über die im Ausführungsbeispiel dargestellte Kabelverbindung auch drahtlos erfolgen. Auch die Auslösung der Klickreizfolge und der Meßwerterfassung durch Betätigung eines Schalters an der Elektrodeneinheit kann auf drahtlosem Wege erfolgen.

## Patentansprüche

1. Gerät zur Ableitung akustisch evozierter Gehirnpotentiale bei der Hirnstamm-Audiometrie, mit einer Anzahl von am Kopf des Probanden anzuordnenden Elektroden (20, 21), die mindestens eine Ableitungselektrode (21) und eine an einer davon entfernten Stelle am Kopf anzuordnende Bezugselektrode (22) umfassen, und wobei an einem relativ starren Rahmen (10) ein elektroakustischer wandler (60) angeordnet ist, der als Reizgeber zur akustischen stimulation des Ohres dient, **dadurch gekennzeichnet, daß** die Elektroden (21, 22) an dem Rahmen (10, 11, 12) in definierter räumlicher Orientierung relativ zueinander gehaltert sind, der das Halten des Geräts am Probandenkopf unter gleichzeitiger Anlage aller Elektroden an den entsprechenden Ableitungspunkten ermöglicht.

2. Gerät nach Anspruch 1, **dadurch gekennzeichnet, daß** der elektroakustische Schallwandler ein Lautsprecher (60), der in einer gegen Umgebungsgeräusche gedämpften Hörkapsel (30) untergebracht ist, ein Knochenleitungsschallgeber oder eine Kombination hiervon ist.

3. Gerät nach Anspruch 2, **dadurch gekennzeichnet, daß** in der Hörkapsel (30) außerdem ein Mikrofon (70) untergebracht ist, welches den Schalldruck in der Hörkapsel mißt.

4. Gerät nach Anspruch 2 oder 3, **dadurch gekennzeichnet, daß** die Hörkapsel (30) drehbar am Rahmen (10) befestigt ist, um die relative Orientierung von Hörkapsel und Elektroden für den Gebrauch des Geräts am linken Ohr bzw. am rechten Ohr des Probanden jeweils richtig einstellen zu können.

5. Gerät nach einem der Ansprüche 2 bis 4, **dadurch gekennzeichnet, daß** die Hörkapsel (30) mindestens teilweise aus transparentem Material besteht.

6. Gerät nach einem der Ansprüche 1 bis 5, **dadurch gekennzeichnet daß** die am Rahmen gehalterten Elektroden drei Elektroden umfassen, von welchen eine Elektrode (21) für eine Potentialableitung im Scheitelbereich vorgesehen und an einem entsprechenden Rahmenarm (12) angeordnet ist, eine zweite Elektrode (21) zur Potentialableitung an einem hinter dem Ohr gelegenen Kopfbereich angeordnet und die dritte Elektrode (22) als Bezugselektrode zur Anlage an einem vorderhalb des Ohres gelegenen Kopfbereich am Rahmen angeordnet ist.

7. Gerät nach einem der Ansprüche 1 bis 6, **dadurch gekennzeichnet daß** die Elektroden (21, 22) an dem Rahmen (10, 11, 12) in einer etwa linienförmigen Anordnung angeordnet sind.

8. Gerät nach einem der Ansprüche 1 bis 7, **dadurch gekennzeichnet, daß** an dem Rahmen (10, 11, 12) auch ein EEG-Verstärker (40) angeordnet ist.

9. Gerät nach Anspruch 8, **dadurch gekennzeichnet, daß** der EEG-Verstärker (40) mit seinem Gehäuse als Griffstück für den Rahmen (10, 11, 12) ausgebildet ist.

10. Gerät nach Anspruch 8 oder 9 dadurch bekennzeichnet, daß auch eine komplette Meßeinrichtung zum Messen der abgeleiteten Gehirnpotentiale an dem Rahmen (10, 11, 12) angeordnet ist.

11. Gerät nach Anspruch 9 oder 10, **dadurch gekennzeichnet, daß** an dem Rahmen (10) auch eine Einrichtung zur drahtlosen Übertragung der erfaßten Signale bzw. der Meßwerte zu einer Signalgeber/Auswerteeinheit (1) vorgesehen ist.

12. Gerät nach einem der Ansprüche 1 bis 11, **dadurch gekennzeichnet, daß** an dem Rahmen (10) ein fingerbetätigbarer Schalter (6) zum Auslösen der Erzeugung akustischer Reizsignale und der Meßwerterfassung vorgesehen ist.

## Claims

1. Device for the derivation of acoustically evoked cerebral potentials in brain stem audiometry, with a number of electrodes (20, 21) for arrangement at the head of the subject, comprising at least a derivation electrode (21) and, to be arranged away from this at the head, a reference electrode (22), with arranged on a relatively rigid frame (10) an electro-acoustic transducer (60) serving as an exciter for acoustic stimulation of the ear, **characterised in that** the electrodes (21, 22) are mounted on the frame (10, 11, 12) in a defined spatial orientation relative to one another, which frame enabling the location of the device at the subject's head while at the same time locating all electrodes at the corresponding derivation points.

2. Device according to Claim 1, **characterised in that** the electro-acoustic transducer is a loud speaker (60) which is housed in an earpiece (30) insulated against ambient noise, a bone conduction vibrator or a combination of both.

3. Device according to Claim 2, **characterised in that** housed in the earpiece (30) is also a microphone (70) that measures the sound pressure in the earpiece.

4. Device according to Claim 2 or 3, **characterised in that** the earpiece (30) is pivot-mounted on the frame (10) in order to allow correct adjustment of the relative orientation of earpiece and electrodes for use of the device either on the left ear or the right ear of the subject.

5. Device according to one of Claims 2 to 4, **characterised in that** the earpiece (30) consists at least partially of transparent material.

6. Device according to one of Claims 1 to 5, **characterised in that** the electrodes mounted on the frame comprise three electrodes of which one electrode (21) is provided for potential derivation in the crown area and is arranged on a corresponding frame arm (12), a second electrode (21) is located for potential derivation at an area of the head located behind the ear, and the third electrode (22) as a reference electrode is arranged on the frame for location at a head area located in front of the ear.

7. Device according to one of Claims 1 to 6, **characterised in that** the electrodes (21, 22) are arranged on the frame (10, 11, 12) in an approximately linear-shaped arrangement.

8. Device according to one of Claims 1 to 7, **characterised in that** also arranged on the frame (10, 11, 12) is an EEG amplifier (40).

9. Device according to Claim 8, **characterised in that** the EEG amplifier (40) is designed with its housing as a grip for the frame (10, 11, 12).

10. Device according to Claim 8 or 9, **characterised in that** a complete measuring apparatus for measuring the derived cerebral potentials is also arranged on the frame (10, 11, 12).

11. Device according to Claim 9 or 10, **characterised in that** also provided on the frame (10) is a device for wireless transmission of the recorded signals or measured values to a transducing sensor/evaluation unit (1).

12. Device according to one of Claims 1 to 11, **characterised in that** provided on the frame (10) is a finger-operable switch (6) for initiating the generation of acoustic excitation signals and measured value acquisition.

## Revendications

1. Dispositif pour la dérivation de potentiels cérébraux acoustiquement provoqués lors de l'audiométrie du tronc cérébral, comportant un nombre d'électrodes (20, 21) à disposer sur la tête du sujet, qui comportent au moins une électrode de dérivation (21) et une électrode de référence (22) à disposer à un endroit éloigné sur la tête par rapport à la première, un convertisseur électroacoustique (60) étant disposé sur un cadre relativement rigide (10), lequel convertisseur servant d'excitateur pour la stimulation acoustique de l'oreille, **caractérisé en ce que** les électrodes (21, 22) sont fixées au cadre (10, 11, 12) réciproquement dans une orientation spatiale définie qui permet le maintien du dispositif sur la tête du sujet avec l'application simultanée de toutes les électrodes aux points de dérivation correspondants.

2. Dispositif selon la revendication 1, **caractérisé en ce que** le convertisseur électroacoustique est un haut-parleur (60), qui est logé dans une capsule auriculaire (30) isolée contre les bruits ambiants, un vibreur à conduction par les os ou une combinaison de ceux-ci.

3. Dispositif selon la revendication 2, **caractérisé en ce que** dans la capsule auriculaire (30) un microphone (70) est logé par ailleurs, qui mesure la pression du son dans la capsule auriculaire.

4. Dispositif selon les revendications 2 ou 3, **caractérisé en ce que** la capsule auriculaire (30) est fixée de manière à pouvoir pivoter dans le cadre (10) afin de pouvoir correctement régler l'orientation relative de la capsule auriculaire et des électrodes pour l'usage du dispositif sur l'oreille gauche et sur l'oreille droite du sujet.

5. Dispositif selon l'une quelconque des revendications 2 à 4, **caractérisé en ce que** la capsule auriculaire (30) consiste au moins partiellement en matière transparente.

6. Dispositif selon l'une quelconque des revendications 1 à 5, **caractérisé en ce que** les électrodes fixées au cadre comportent trois électrodes dont une électrode (21) est prévue pour la dérivation de potentiels cérébraux dans la zone du sommet et est disposée sur un bras de cadre (12) correspondant, une seconde électrode (21) est disposée pour la dérivation des potentiels dans une zone de la tête située derrière l'oreille et la troisième électrode (22) est disposée sur le cadre en tant qu'électrode de référence pour être placée dans une zone de la tête située devant l'oreille.

7. Dispositif selon l'une quelconque des revendications 1 à 6, **caractérisé en ce que** les électrodes (21, 22) sont disposées sur le cadre (10, 11, 12) dans une disposition approximativement linéaire.

8. Dispositif selon l'une quelconque des revendications 1 à 7, **caractérisé en ce qu'**un amplificateur EEG (40) peut également être disposé sur le cadre (10, 11, 12).

9. Dispositif selon la revendication 8, **caractérisé en ce que** l'amplificateur EEG (40) avec son boîtier est formé en tant que poignée pour le cadre (10, 11, 12).

10. Dispositif selon les revendications 8 à 9, **caractérisé en ce qu'**un dispositif de mesure complet pour mesurer les potentiels cérébraux dérivés est également disposé sur le cadre (10, 11, 12).

11. Dispositif selon les revendications 9 ou 10, **caractérisé en ce qu'**un dispositif pour la transmission sans fil des signaux et des valeurs de mesure saisis à un émetteur de signaux/une unité de dépouillement (1) est prévu.

12. Dispositif selon l'une quelconque des revendications 1 à 11, **caractérisé en ce qu'**un interrupteur (6) pouvant être actionné par un doigt pour déclencher la production de signaux d'excitation acoustiques et la saisie des valeurs de mesure est prévu.
